# EUROPÄISCHE PATENTSCHRIFT

(11) **EP 1 895 327 B1**
(45) Veröffentlichungstag und Bekanntmachung des Hinweises auf die Patenterteilung: **11.11.2009**
(21) Anmeldenummer: 06125512.1
(22) Anmeldetag: 06.12.2006
(51) Int. Cl.: G01T 1/29

(54) **Löschen einer Speicherleuchtstoffschicht**
Erasing of a phosphor imaging plate
Effacement d'une couche d'enregistrement luminescente

(30) Priorität: 31.08.2006 EP 06119938
(43) Veröffentlichungstag der Anmeldung: 05.03.2008
(73) Patentinhaber: Agfa HealthCare NV, 2640 Mortsel (BE)
(72) Erfinder: Bode, Andreas, Dr., 81476 München (DE); Reiser, Georg, Dr., 80337 München (DE); Weber, Marc, Dr., 81679 München (DE); Thoma, Ralph, Dr., 86167 Augsburg (DE); Stallmeister, Stefan, 85625 Glonn (DE); Baunach, Stephan, 80339 München (DE); Minwegen, Heike, 83043 Bad Aibling (DE); Fasbender, Robert, Dr., 91054 Erlangen (DE); Dedler, Rupert, 86860 Jengen-Weinhausen (DE)
(74) Vertreter: Linsmeier, Josef

(56) Entgegenhaltungen:
- EP-A1- 0 704 716
- WO-A-01/50960
- US-A1- 5 534 709
- US-A1- 2003 123 613
- US-A1- 2005 017 207

## Beschreibung

Die vorliegende Erfindung betrifft eine Vorrichtung zum Löschen einer Speicherleuchtstoffschicht gemäß dem Oberbegriff von Anspruch 1 sowie ein entsprechendes System mit einer solchen Vorrichtung und einer Speicherleuchtstoffschicht.

Eine solche Vorrichtung und ein solches System werden insbesondere im Bereich der Computer-Radiografie (CR) für medizinische Zwecke eingesetzt. Von einem Objekt, beispielsweise einem Patienten oder einem Körperteil des Patienten, wird mittels Röntgenbestrahlung ein Bild erzeugt, das in einer Speicherleuchtstoffschicht als latentes Bild abgespeichert wird. Ein solches Röntgenbild enthält somit Röntgeninformationen über das Objekt. Zum Auslesen der in der Speicherleuchtstoffschicht abgespeicherten Röntgeninformationen wird die Speicherleuchtstoffschicht mittels einer Bestrahlungseinrichtung angeregt. Die Speicherleuchtstoffschicht emittiert aufgrund dieser Anregung Strahlung, die eine Intensität entsprechend der in der Speicherleuchtstoffschicht abgespeicherten Röntgeninformationen aufweist. Die von der Speicherleuchtstoffschicht ausgesandte Strahlung wird von einer Detektionseinrichtung erfasst und in elektrische Signale gewandelt, die ein Abbild der Röntgeninformationen enthalten. Die elektrischen Signale werden weiterverarbeitet und die in der Speicherleuchtstoffschicht abgespeicherten Röntgeninformationen anschließend sichtbar gemacht. Die Röntgeninformationen können beispielsweise direkt auf einem Monitor dargestellt oder mittels eines speziell für Röntgenbilder verwendbaren Druckers auf einen fotografischen Röntgenfilm geschrieben werden.

Nach dem Auslesen der Röntgeninformationen aus der Speicherleuchtstoffschicht verbleiben in dieser Reste des latenten Bildes. Ferner können Rauschinformationen abgespeichert sein. Um die Speicherleuchtstoffschicht für weitere Röntgenaufnahmen verwenden zu können, wird sie daher gelöscht. Dazu wird eine Strahlungsquelle verwendet, die eine Löschstrahlung auf die Speicherleuchtstoffschicht ausgibt. Aus der US 7,057,200 B2 ist eine Vorrichtung zum Löschen einer Speicherleuchtstoffschicht bekannt. Diese Löschvorrichtung enthält als Strahlungsquelle zum Aussenden der Löschstrahlung zwei parallel zueinander angeordnete Zeilen mit Leuchtdioden, die auf Kühlkörpern aus Aluminium angeordnet sind. Zum Löschen wird die Speicherleuchtstoffschicht in eine Transportrichtung durch einen Strahlengang der Leuchtdiodenzeilen geschoben. Die beiden Leuchtdiodenzeilen sind in Reflektoren integriert, die voneinander beabstandet sind. Die Reflektoren dienen dazu, von den Leuchtdioden ausgegebene Löschstrahlung in Richtung der Speicherleuchtstoffschicht zu reflektieren. Die Reflektoren werden jeweils mittels zweier Reflektorflächen gebildet, die in Transportrichtung zu beiden Seiten der Leuchtdiodenzeilen angeordnet sind. Die Reflektorflächen grenzen mit stumpfen Innenwinkeln an die Kühlkörper, so dass sich die Reflektoren ausgehend von den Kühlkörpern in Richtung der Speicherleuchtstoffschicht öffnen.

Es ist die Aufgabe der vorliegenden Erfindung, eine hohe Effizienz beim Löschen einer Speicherleuchtstoffschicht zu ermöglichen.

Diese Aufgabe wird gemäß der technischen Lehre des Anspruchs 1 oder des Anspruchs 22 gelöst.

Bei der erfindungsgemäßen Vorrichtung ist vorgesehen, dass der Reflektor derart angeordnet und/oder ausgestaltet ist, dass er Löschstrahlung, welche von der Speicherleuchtstoffschicht reflektiert wird, wieder in Richtung auf die Speicherleuchtstoffschicht reflektiert, und die Strahlungsquelle auf einem Sockel angeordnet ist, wobei der Sockel näher an der Aufnahmeebene angeordnet ist als wenigstens ein Teil des Reflektors. Das erfindungsgemäße System enthält die erfindungsgemäße Vorrichtung und eine Speicherleuchtstoffschicht. Vorteilhafte Ausgestaltungen der Erfindung können den abhängigen Ansprüchen entnommen werden.

Der Erfindung liegt die Erkenntnis zugrunde, dass die Speicherleuchtstoffschicht einen hohen Reflexionsgrad hat, wodurch ein großer Teil der Löschstrahlung, mit welcher die Speicherleuchtstoffschicht bestrahlt wird, von dieser ungenutzt reflektiert wird und dadurch nicht zum Löschen von in der Speicherleuchtstoffschicht abgespeicherten, unerwünschten Bildinformationen beiträgt.

Erfindungsgemäß wird die von der Speicherleuchtstoffschicht reflektierte Löschstrahlung von dem Reflektor aufgefangen und erneut in Richtung der Speicherleuchtstoffschicht zurückreflektiert. Diese Reflexion kann gerichtet oder diffus sein. Der Reflektor ist diesbezüglich in seiner Form und Größe geeignet ausgestaltet und/oder von der Speicherleuchtstoffschicht beabstandet.

Die von dem Reflektor zurückreflektierte Löschstrahlung kann somit ebenfalls zum Löschen der Speicherleuchtstoffschicht beitragen. Dadurch wird der Wirkungsgrad des Löschens wesentlich verbessert. Ferner ist der Leistungsbedarf geringer, was zu weniger Verlustwärme und zu einer Erhöhung der Lebensdauer führt.

Aufgrund der erfindungsgemäßen Anbringung der Strahlungsquelle auf einem Sockel wird darüber hinaus gewährleistet, dass sehr wenig der von dem Reflektor reflektierten Löschstrahlung, die zuvor bereits von der Speicherleuchtstoffschicht in Richtung des Reflektors reflektiert wurde, durch den Reflektor in die Strahlungsquelle hinein reflektiert wird. Dadurch wird eine unerwünschte Reabsorption von Löschstrahlung, welche von der Speicherleuchtstoffschicht und vom Reflektor reflektiert wird, in der Strahlungsquelle vermieden, so dass Effizienzverluste stark vermindert werden.

Der Sockel ist vorzugsweise ein Bestandteil des Reflektors und wird durch eine Erhöhung des Reflektors in Richtung der Aufnahmeebene gebildet. Vorzugsweise wird der Reflektor zusammen mit dem Sockel aus einem Stück, insbesondere einem reflektierenden Blech, geformt. Die eigentlichen Reflektorflächen des Reflektors grenzen hierbei vorteilhafterweise unmittelbar an den Sockel an.

Die Strahlungsquelle weist vorzugsweise eine Vielzahl von einzelnen Lichtquellen, wie z.B. Leuchtdioden, auf, die in einer Strahlungsebene angeordnet sind, die parallel zur Aufnahmeebene verläuft.

In einer vorteilhaften Ausgestaltung der Erfindung ist der Sockel auf der der Aufnahmeebene zugewandten Seite reflektierend ausgestaltet. Dadurch kann gewährleistet werden, dass der Sockel ebenfalls von der Speicherleuchtstoffschicht reflektierte Löschstrahlung erneut in Richtung der Speicherleuchtstoffschicht reflektiert. Dies erhöht noch weiter die Effizienz beim Löschen der Speicherleuchtstoffschicht.

In einer weiteren vorteilhaften Ausgestaltung ist der Sockel insbesondere in Richtung der Aufnahmeebene gewölbt ausgestaltet. Der Sockel hat eine, bezogen auf die Aufnahmeebene, konvexe Ausgestaltung. In einer besonders bevorzugten Ausgestaltung der Erfindung weist der Sockel wenigstens eine Vertiefung auf, in der die Strahlungsquelle ausgebildet ist. Besonders vorteilhaft ist der Sockel näher als der gesamte Reflektor an der Aufnahmeebene angeordnet. Diese vorteilhaften Ausgestaltungen ermöglichen jede für sich, und insbesondere gemeinsam, einen besonders guten Schutz der Strahlungsquelle vor von dem Reflektor reflektierter Löschstrahlung.

Bevorzugt weist der Reflektor eine von der Aufnahmeebene weg gewölbte Reflektorfläche auf. Der Reflektor hat eine, bezogen auf die Aufnahmeebene, konkave Ausgestaltung. Dadurch kann die reflektierte Löschstrahlung besonders gut gerichtet in Richtung der Speicherleuchtstoffschicht reflektiert werden, ohne dabei auf die Strahlungsquelle zu treffen.

In einer vorteilhaften Ausgestaltung der Erfindung weist der Reflektor eine ebene Reflektorfläche auf, die insbesondere parallel zur Aufnahmeebene verläuft. Eine solche Reflektorform kann zuverlässig reflektierte Löschstrahlung aufsammeln und zur Speicherleuchtstoffschicht zurückreflektieren. Diese Reflektorform ist kostengünstig herstellbar und kompakt ausgestaltbar.

In einer weiteren vorteilhaften Ausgestaltung weist der Reflektor eine Reflektorfläche mit einer Struktur auf. Mit einer solchen Struktur kann die Effizienz des Löschens noch weiter erhöht werden. Die Struktur kann insbesondere geriffelt, dach-, sägezahn- und/oder dreieckförmig, etc., sein.

In einer besonders bevorzugten Ausgestaltung der Erfindung ist die strukturierte Reflektorfläche retroreflektiv ausgestaltet, so dass sie zumindest einen Teil der Löschstrahlung an Stellen der Speicherleuchtstoffschicht zurückreflektiert, an denen sie von der Speicherleuchtstoffschicht zuvor reflektiert wurde. Eine solche retroreflektive Reflektorfläche gewährleistet ein besonders gleichmäßiges Löschen der Speicherleuchtstoffschicht. An diejenigen Stellen, die viel Löschstrahlung reflektiert haben, wird auch viel Löschstrahlung zurückreflektiert. Die retroreflektive Reflektorfläche kann insbesondere nach Art eines sogenannten "Katzenauges" ausgestaltet sein und als Folie eingesetzt werden. Dies ist besonders platzsparend und kostengünstig.

Besonders bevorzugt ist ein Antrieb zum Erzeugen einer Relativbewegung zwischen der Aufnahmeebene und der Strahlungsquelle vorhanden. Dies ermöglicht auf einfache Weise ein gleichmäßiges Erzeugen der Relativbewegung und ein effizientes Löschen der Speicherleuchtstoffschicht.

Besonders vorteilhaft weist der Reflektor wenigstens zwei Reflektorflächen auf, so dass der Reflektor, in Richtung der Relativbewegung betrachtet, zu beiden Seiten der Strahlungsquelle ausgebildet ist. Dadurch kann besonders viel Löschstrahlung erfasst und zurückreflektiert werden.

In einer besonders bevorzugten Ausgestaltung der Erfindung ist der Reflektor in Richtung der Relativbewegung spiegelsymmetrisch ausgestaltet, wobei eine Symmetrieachse senkrecht zur Richtung der Relativbewegung und, in Richtung der Relativbewegung betrachtet, zentral durch die Strahlungsquelle hindurch verläuft. Durch einen solchen Reflektor kann zu beiden Seiten der Strahlungsquelle eine große Menge an reflektierter Löschstrahlung erfasst und zur Speicherleuchtstoffschicht zurückreflektiert werden.

In einer vorteilhaften Ausgestaltung der Erfindung ist eine Ausbreitung des Reflektors in Richtung der Relativbewegung wenigstens zehnmal so groß wie ein kleinster Abstand des Reflektors von der Aufnahmeebene. Durch diese Dimensionierung des Reflektors mit einer großen Ausbreitung in Richtung der Relativbewegung und einem kleinen Abstand zu der Aufnahmeebene für die Speicherleuchtstoffschicht kann insbesondere gewährleistet werden, dass ein großer Teil der von der Speicherleuchtstoffschicht reflektierten oder gestreuten Löschstrahlung aufgefangen oder erfasst und erneut in Richtung der Speicherleuchtstoffschicht reflektiert werden kann.

Besonders vorteilhaft weist die Strahlungsquelle wenigstens zwei senkrecht zur Richtung der Relativbewegung und parallel zu der Aufnahmeebene verlaufende Zeilen mit Leuchtdioden auf. Dadurch lässt sich eine ausreichend hohe Intensität von Löschstrahlung erzeugen, wobei der Leistungsverbrauch der Leuchtdioden besonders gering ist.

Bevorzugt sind die wenigstens zwei Zeilen mit Leuchtdioden in den Sockel integriert. Ein Abstand zwischen den wenigstens zwei Zeilen ist kleiner oder gleich einem Abstand der Leuchtdioden zu der Aufnahmeebene. Die Löschvorrichtung kann dadurch besonders kompakt ausgestaltet werden. Ferner kann die von den Leuchtdiodenzeilen emittierte Löschstrahlung besonders gut ausgerichtet auf die Speicherleuchtstoffschicht ausgegeben werden.

Besonders bevorzugt sind den wenigstens zwei Zeilen mit Leuchtdioden jeweils ein eigener Reflektor und jeweils ein eigener Sockel zugeordnet. Die Leuchtdioden der jeweiligen Zeilen emittieren ferner eine Strahlung in einem anderen, insbesondere schmalbandigen, Wellenlängenbereich als die Leuchtdioden der anderen Zeilen. Die Reflektoren sind insbesondere so ausgestaltet, dass sie zu einer Trennung der von den unterschiedlichen Leuchtdiodenzeilen ausgegebenen Löschstrahlungen mit den unterschiedlichen Wellenlängenbereichen beitragen. Dadurch kann verhindert werden, dass sich bestimmte Spektralbereiche gegenseitig beeinflussen oder stören. Die Wellenlängenbereiche können vorteilhafterweise so gewählt sein, dass solche Wellenlängen, die nicht zum Löschen des eingesetzten Typs von Speicherleuchtstoffschicht beitragen, nicht vorhanden sind. Dadurch ist ein sonst erforderliches Herausfiltern solcher Wellenlängen nicht erforderlich. Ferner wird eine besonders gute Löscheffizienz erreicht.

Vorzugsweise sind die wenigstens zwei Zeilen mit Leuchtdioden in Richtung der Relativbewegung so hintereinander angeordnet, dass beim Ausführen der Relativbewegung zum Löschen der Speicherleuchtstoffschicht eine kurzwellige Löschstrahlung vor einer langwelligen Löschstrahlung auf die Speicherleuchtstoffschicht trifft. Dabei kann insbesondere blaue Löschstrahlung vor roter Löschstrahlung auf die Speicherleuchtstoffschicht gerichtet sein. Auf diese Weise ist eine besonders gute Löscheffizienz gewährleistet.

Bevorzugt ist eine Intensität der langwelligen Löschstrahlung größer als eine Intensität der kurzwelligen Löschstrahlung. Insbesondere kann ein Verhältnis von blauer zu roter Löschstrahlung so gewählt sein, dass 66% der Löschstrahlung rote und 33% der Löschstrahlung blaue Löschstrahlung ist. Dies gewährleistet eine noch bessere Löscheffizienz.

Besonders bevorzugt ist eine dem Reflektor, in Richtung senkrecht zur Richtung der Relativbewegung betrachtet, gegenüberliegende, weitere reflektierende Fläche zum Reflektieren von Löschstrahlung vorhanden. Die weitere reflektierende Fläche kann so ausgestaltet sein, dass sie die Löschstrahlung gerichtet oder diffus reflektiert. Durch diese weitere reflektierende Fläche kann vorteilhafterweise zu Beginn und/oder am Ende des Löschvorgangs, d. h. wenn der Strahlengang der Strahlenquelle nicht oder nicht vollständig auf die Speicherleuchtstoffschicht gerichtet ist, die von der Strahlungsquelle ausgegebene Löschstrahlung von der weiteren reflektierenden Fläche in Richtung des Reflektors reflektiert werden. Die von der weiteren reflektierenden Fläche reflektierte Löschstrahlung, die die Speicherleuchtstoffschicht insbesondere noch nicht erreicht hat, kann somit von dem Reflektor auf die Speicherleuchtstoffschicht gerichtet werden. Insbesondere die vordere und ggf. die hintere Kante der Speicherleuchtstoffschicht können somit mit hoher Effizienz gelöscht werden.

Vorzugsweise ist die weitere reflektierende Fläche auf der dem Reflektor abgewandten Seite der Aufnahmeebene angeordnet. Dadurch kann besonders gut gewährleistet werden, dass nicht auf die Speicherleuchtstoffschicht auftreffende Löschstrahlung von der weiteren reflektierenden Fläche reflektiert wird, um dann von dem Reflektor in Richtung der Speicherleuchtstoffschicht reflektiert zu werden.

Vorzugsweise sind Stirnflächen und/oder Seitenflächen des Reflektors reflektierend ausgestaltet. Dadurch kann von der Speicherleuchtstoffschicht reflektierte Löschstrahlung noch besser und effizienter erfasst und in Richtung der Speicherleuchtstoffschicht zurück reflektiert werden.

Die Speicherleuchtstoffschicht des erfindungsgemäßen Systems weist vorzugsweise einen Reflexionsgrad für die Löschstrahlung von größer oder gleich 70%, insbesondere größer oder gleich 80%, auf. Gerade für Speicherleuchtstoffschichten mit einem solch hohen Reflexionsgrad kann die erfindungsgemäße Löschvorrichtung besonders effizient eingesetzt werden.

Weitere Vorteile der Erfindung ergeben sich aus der folgenden Beschreibung von Ausführungsbeispielen, wobei Bezug auf die beigefügten Zeichnungen genommen wird. Es zeigen:
- Fig. 1: ein erstes Ausführungsbeispiel einer erfindungsgemäßen Löschvorrichtung mit einem Reflektor, der ebene, parallel zu einer Speicherleuchtstoffschicht verlaufende Reflektorflächen aufweist, und mit einer dem Reflektor gegenüberliegenden weiteren Reflexionsfläche,
- Fig. 2: eine perspektivische Darstellung der Löschvorrichtung gemäß dem ersten Ausführungsbeispiel,
- Fig. 3: ein zweites Ausführungsbeispiel der erfindungsgemäßen Löschvorrichtung mit einem Reflektor, der Reflektorflächen mit einer dreieckförmigen Struktur aufweist,
- Fig. 4: eine Draufsicht auf eine Unterseite der Strahlungsquelle der Löschvorrichtung gemäß dem zweiten Ausführungsbeispiel,
- Fig. 5: eine perspektivische Darstellung der Löschvorrichtung gemäß dem zweiten Ausführungsbeispiel,
- Fig. 6: ein drittes Ausführungsbeispiel der erfindungsgemäßen Löschvorrichtung mit einem rinnenförmigen Reflektor, der von der Aufnahmeebene für die Speicherleuchtstoffschicht weg gewölbte Reflektorflächen aufweist,
- Fig. 7: eine perspektivische Darstellung der Löschvorrichtung gemäß dem dritten Ausführungsbeispiel,
- Fig. 8: ein viertes Ausführungsbeispiel der erfindungsgemäßen Löschvorrichtung mit einem Reflektor mit dreieckförmig strukturierten Reflektorflächen,
- Fig. 9: ein fünftes Ausführungsbeispiel der erfindungsgemäßen Löschvorrichtung mit einem Reflektor, der strukturierte Reflektorflächen mit einer Vielzahl kleiner Dreiecke aufweist,
- Fig. 10: ein sechstes Ausführungsbeispiel der erfindungsgemäßen Löschvorrichtung mit einem Reflektor, der ebene Reflektorflächen und einen in Richtung der Aufnahmeebene für die Speicherleuchtstoffschicht gewölbten Sockel aufweist, und
- Fig. 11: ein siebtes Ausführungsbeispiel der erfindungsgemäßen Löschvorrichtung mit zwei Leuchtdiodenzeilen, die Licht in verschiedenen Wellenlängenbereichen ausgeben und jeweils einen eigenen Reflektor aufweisen.

Im Folgenden werden, sofern nicht anders angegeben, für gleiche oder gleichwirkende Elemente gleiche Bezugszeichen verwendet.

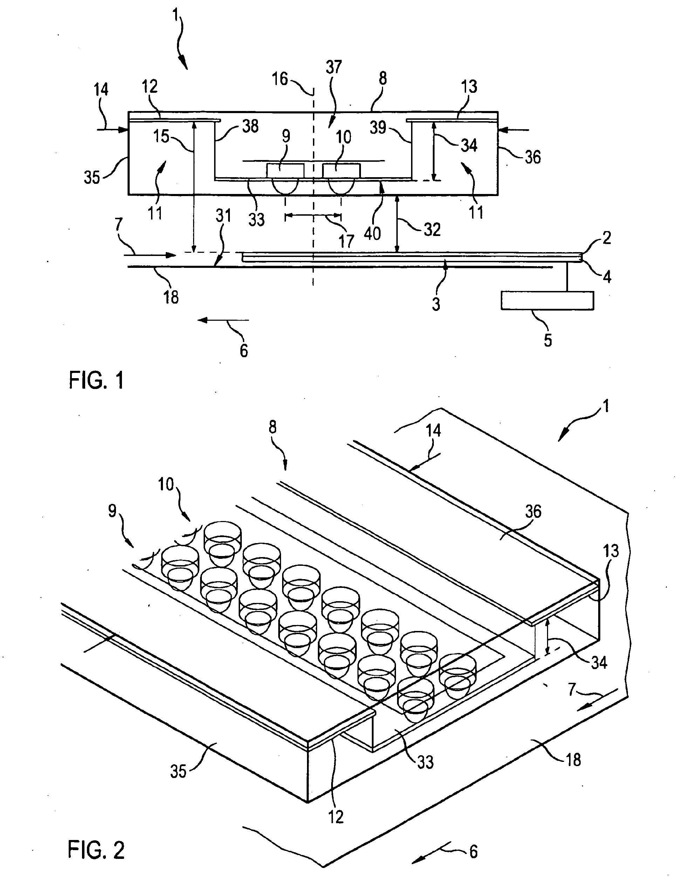

Fig. 1 zeigt ein erstes Ausführungsbeispiel einer erfindungsgemäßen Löschvorrichtung 1 zum Löschen von Röntgeninformationen, die in einer Speicherleuchtstoffschicht 2 einer Speicherleuchtstoffplatte 3 abgespeichert sind. Die Speicherleuchtstoffplatte 3 weist eine Trägerschicht 4 auf, auf der die Speicherleuchtstoffschicht 2 aufgebracht ist. Die Speicherleuchtstoffschicht 2 setzt sich vorzugsweise aus einer Vielzahl von Speicherleuchtstoffpartikeln zusammen, die zum Speichern der Röntgeninformationen dienen. Die Trägerschicht 4 hat vorzugsweise eine Dicke von 1-2 mm. Die Speicherleuchtstoffplatte 3 ist hier nicht Teil der Löschvorrichtung 1, sondern kann von außen in die Löschvorrichtung 1 hinein geschoben werden. Innerhalb der Löschvorrichtung 1 wird die Speicherleuchtstoffplatte 3 mittels eines Antriebs 5 in eine Transportrichtung 6 verschoben, die durch einen Pfeil angezeigt wird. Die Speicherleuchtstoffplatte 3 ist in der Löschvorrichtung 1 in einer Aufnahmeebene 7 geführt und in dieser Aufnahmeebene 7 verschiebbar. Unterhalb der Aufnahmeebene 7 befindet sich eine Auflage 18, auf der die Speicherleuchtstoffplatte 3 aufliegen und verschiebbar geführt werden kann.

Die Löschvorrichtung 1 enthält eine Strahlungsquelle 8 zum Aussenden von Löschstrahlung. Die Strahlungsquelle 8 weist hier zwei parallel zueinander angeordnete Leuchtdiodenzeilen 9 und 10 auf. Die Leuchtdiodenzeilen 9, 10 enthalten jeweils eine Vielzahl von nebeneinander angeordneten Leuchtdioden. Die Leuchtdiodenzeilen 9, 10 erstrecken sich über die gesamte Länge der Speicherleuchtstoffschicht 2. Die Länge der Speicherleuchtstoffschicht 2 verläuft in der Darstellung nach Fig. 1 senkrecht zur Transportrichtung 6 und in Richtung der Zeichnungsblattebene. Die Breite der Speicherleuchtstoffschicht 2 erstreckt sich in Transportrichtung 6. Die Leuchtdiodenzeilen 9, 10 sind auf einem ebenen Sockel 33 angeordnet, der sich parallel zu der Aufnahmeebene 7 erstreckt.

Mittels des Antriebs 5 wird die Speicherleuchtstoffschicht 2 in Transportrichtung 6 mit gleichförmiger Transportgeschwindigkeit an den Leuchtdiodenzeilen 9, 10 vorbeitransportiert. Dadurch durchläuft die Speicherleuchtstoffschicht 2 die Strahlengänge der Leuchtdiodenzeilen 9, 10. Es ist alternativ auch möglich, anstelle der Speicherleuchtstoffplatte 3 die Strahlungsquelle zu transportieren, wobei dann die Speicherleuchtstoffplatte 3 in der Löschvorrichtung 1 nicht bewegt wird. In beiden Fällen wird zwischen der Strahlungsquelle 8 und der in der Aufnahmeebene 7 liegenden Speicherleuchtstoffschicht 2 eine Relativbewegung ausgeführt, die hier in Richtung des Pfeils für die Transportrichtung 6 verläuft.

Beim Transportieren der Speicherleuchtstoffplatte 3 trifft das von den Leuchtdioden der Leuchtdiodenzeilen 9, 10 emittierte Löschlicht auf die Speicherleuchtstoffschicht 2. Ein Teil des Löschlichts dringt in die Speicherleuchtstoffschicht 2 ein und löscht die in ihr nach einem Auslesen verbliebenen Röntgeninformationen und ein gegebenenfalls vorhandenes Rauschen. Da die Speicherleuchtstoffschicht 2 für das Löschlicht einen Reflexionsgrad von wenigstens 70%, insbesondere von wenigstens 80%, aufweist, wird ein großer Teil des Löschlichts von der Speicherleuchtstoffschicht 2 reflektiert, ohne zum Löschen beizutragen.

Um eine hohe Effizienz und einen hohen Wirkungsgrad beim Löschen zu erzielen, hat die Löschvorrichtung 1 einen Reflektor 11. Der Reflektor 11 weist im vorliegenden Ausführungsbeispiel zwei ebene, parallel zu der Aufnahmeebene 7 und der Speicherleuchtstoffschicht 2 verlaufende Reflektorflächen 12 und 13 auf. Die Reflektorflächen 12, 13 sind, in Transportrichtung 6 betrachtet, zu beiden Seiten des Sockels 33 angeordnet und vorteilhafterweise gleich groß. Es ist aber ebenso möglich, nur eine einzige Reflektorfläche an einer der Seiten des Sockels 33 vorzusehen. Ferner ist es möglich, eine der beiden Reflektorflächen 12, 13 kleiner auszugestalten als die andere.

Der Sockel 33 ist erfindungsgemäß um einen Abstand 34 näher an der Aufnahmeebene 7 angeordnet als die Reflektorflächen 12, 13. Der Sockel 33 ist im vorliegenden ersten Ausführungsbeispiel somit näher als der gesamte Reflektor 11 mit seinen Reflektorflächen 12, 13 an der Aufnahmeebene 7 angeordnet. Die Reflektorflächen 12, 13 sind über Verbindungsflächen 38 und 39 mit dem Sockel 33 verbunden.

Die Reflektorflächen 12, 13 und der Sockel 33 erstrecken sich jeweils über die gesamte Länge der Speicherleuchtstoffschicht 2. In Transportrichtung 6 betrachtet, erstrecken sie sich gemeinsam über eine Breite 14 der Strahlungsquelle 8. Von der Oberfläche der in der Aufnahmeebene 7 befindlichen Speicherleuchtstoffschicht 2 weist der Reflektor 11 mit seinen Reflektorflächen 12, 13 einen kleinsten Abstand 15 auf. Die Breite 14 ist wenigstens zehnmal so groß wie der kleinste Abstand 15.

Die Strahlungsquelle 8 und insbesondere der Reflektor 11 sind in Transportrichtung 6 spiegelsymmetrisch ausgestaltet. Dabei verläuft eine Symmetrieachse 16 senkrecht zur Transportrichtung 6 und, bezogen auf die Breite 14, zentral durch die Strahlungsquelle 8 hindurch. Im vorliegenden Ausführungsbeispiel verläuft die Symmetrieachse 16 somit zwischen den beiden Leuchtdiodenzeilen 9, 10.

Die zwei Leuchtdiodenzeilen 9, 10 sind hier zentral in den Sockel 33 integriert. Ein Abstand 17 zwischen den zwei Leuchtdiodenzeilen 9, 10 ist vorteilhafterweise kleiner oder gleich einem Abstand 32 der Leuchtdioden zu der in der Aufnahmeebene 7 liegenden Speicherleuchtstoffschicht 2.

Die Reflektorflächen 12, 13 weisen auf ihren zur Speicherleuchtstoffschicht 2 hin gewandten Oberflächen reflektierende Schichten auf, die für von der Speicherleuchtstoffschicht 2 reflektiertes Löschlicht hochgradig reflektierend sind. Gleiches gilt im vorliegenden Ausführungsbeispiel für den Sockel 33, der auf seiner in Richtung der Aufnahmeebene 7 weisenden Oberfläche mit einer reflektierenden Schicht 40 versehen ist, und die Verbindungsflächen 38, 39. Durch diese reflektierenden Schichten der Reflektorflächen 12, 13 und des Sockels 33 wird Löschlicht, das von der Speicherleuchtstoffschicht 2 reflektiert oder gestreut wird, in Richtung der Speicherleuchtstoffschicht 2 zurückreflektiert. Durch diese Zurückreflexion besteht die Möglichkeit, dass das Löschlicht nun zum Löschen der Röntgeninformationen in die Speicherleuchtstoffschicht 2 eindringt.

Die Strahlungsquelle 8 enthält an ihrer, in Transportrichtung 6 betrachtet, vorderen Stirnseite eine Reflexionsfläche 35 und an ihrer, in Transportrichtung 6 betrachtet, hinteren Stirnseite eine Reflexionsfläche 36. An ihren seitlichen Rändern weist die Strahlungsquelle weitere Reflexionsflächen auf, von denen in der Fig. 1 eine weitere Reflexionsfläche 37 dargestellt ist. Die stirnseitigen und seitlichen Reflexionsflächen 35-37 enthalten insbesondere an ihren nach innen weisenden Oberflächen reflektierende Schichten.

Die einzelnen Leuchtdioden 9, 10 weisen ein Gehäuse auf, welches im dargestellten Ausführungsbeispiel der Fig. 1 durch einen rechteckigen Querschnitt angedeutet ist. Auf dem Gehäuse befindet sich ein für das emittierte Löschlicht transparenter Bereich, welcher im dargestellten Beispiel durch eine rundliche Kuppel dargestellt ist. Zwischen dem Gehäuse und dem transparenten Bereich ist ein lichtemittierender Halbleiter angeordnet. Die einzelnen Leuchtdioden 9, 10 sind vorzugsweise so am Sockel 33 angebracht, dass einerseits das Gehäuse der Leuchtdioden9, 10 durch die auf dem Sockel 33 befindliche reflektierende Schicht 40 von der der Speicherleuchtstoffschicht 2 zugewandten Seite her abgedeckt wird und andererseits der lichtemittierende Halbleiter oberhalb, d. h. auf der der Speicherleuchtstoffschicht 2 zugewandten Seite, der reflektierenden Schicht 40 liegt. Hierdurch wird eine hohe Lichtausbeute der Leuchtdioden 9, 10 bei gleichzeitig hoher Rückreflexion des von der Speicherleuchtstoffschicht 2 reflektierten Löschlichts erreicht.

Die Löschvorrichtung 1 weist eine weitere Reflexionsfläche 31 auf, die den Reflektorflächen 12, 13 und dem Sockel 33, in eine Richtung senkrecht zur Transportgeschwindigkeit 6 betrachtet, gegenüberliegt. Die Reflexionsfläche 31 ist zum Reflektieren von Löschlicht ausgestaltet, das von der Strahlungsquelle 8 ausgegeben wurde. Von der Reflexionsfläche 31 reflektiertes, weiteres Löschlicht wurde ferner gegebenenfalls bereits von der Speicherleuchtstoffschicht 2, den Reflektorflächen 12, 13 und/oder dem Sockel 33 reflektiert. Zum Reflektieren von Löschlicht kann insbesondere auf der der Strahlungsquelle 8 zugewandten Seite der Auflage 18, d. h. auf der dem Reflektor 11 abgewandten Seite der Aufnahmeebene 7, die Reflexionsfläche 31 aufgebracht sein. Die Reflexionsfläche 31 kann vorteilhafterweise als dünne Schicht auf die Auflage 18 aufgebracht sein. Die Reflexionsfläche 31 ist somit derart angeordnet, dass die Speicherleuchtstoffplatte 3 zwischen der Strahlungsquelle 8 und der Reflexionsfläche 31 hindurch transportiert werden kann. Die Reflexionsfläche 31 kann das Löschlicht gerichtet oder diffus zum Reflektor 11 reflektieren. Die Reflexionsfläche 31 ist hier in Transportrichtung 6 vorteilhafterweise so breit wie der Reflektor 11 mit seine Reflektorflächen 12, 13 und der Sockel 33. Dadurch kann vorteilhafterweise gewährleistet werden, dass Formen der Reflexionsfläche 31 und des Reflektors 11 sowie des Sockels 33 besonders gut aufeinander abgestimmt sind. Eine besonders große Menge von Löschstrahlung, welche von der Strahlungsquelle 8 emittiert wird, kann von der Reflexionsfläche 31 reflektiert und eine große Menge dieser reflektierten Löschstrahlung von dem Reflektor 11 und der Schicht 40 des Sockels 33 in Richtung der Speicherleuchtstoffschicht 2 reflektiert werden. Dadurch lässt sich eine besonders gute Löscheffizienz erreichen. Die Fig. 1 zeigt die in die Löschvorrichtung 1 einfahrende Speicherleuchtstoffplatte 3. Die Reflexionsfläche 31 gewährleistet vorteilhafterweise, dass das von der Strahlungsquelle 8 ausgegebene Löschlicht auch dann mit einem hohen Wirkungsgrad zum Löschen beiträgt, wenn die Speicherleuchtstoffplatte 3 sich noch nicht vollständig in der Löschvorrichtung 1 befindet. Insbesondere wird gewährleistet, dass die führende Kante der Speicherleuchtstoffschicht 2 mit erhöhter Effizienz gelöscht wird. Ähnliches gilt, wenn die Speicherleuchtstoffplatte 3 aus der Löschvorrichtung 1 herausfährt. Es ist zum Löschen der Speicherleuchtstoffschicht 2 alternativ möglich, die Speicherleuchtstoffplatte 3 in der Löschvorrichtung 1 ruhen zu lassen und die Strahlungsquelle 8 mitsamt des Reflektors 11 und der gegenüberliegenden Reflexionsschicht 31 entlang der Speicherleuchtstoffplatte 3 zu transportieren.

Fig. 2 zeigt zur Verdeutlichung eine perspektivische Darstellung eines Teils der Löschvorrichtung 1 gemäß Fig. 1. Es ist deutlich die Ausbreitung der Leuchtdiodenzeilen 9 und 10 in Längsrichtung der Löschvorrichtung 1 zu erkennen.

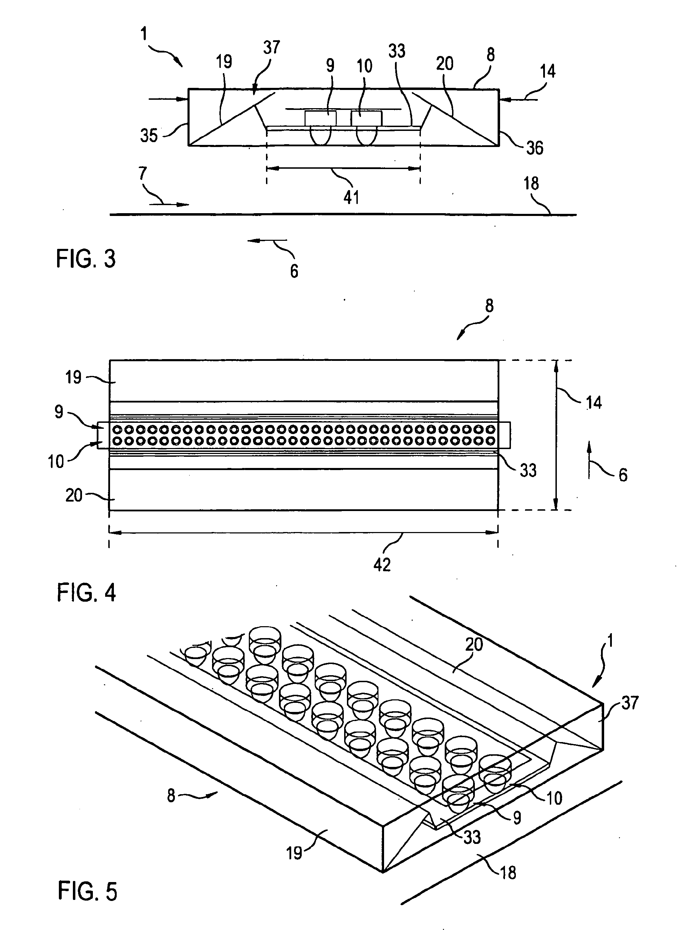

Fig. 3 zeigt ein zweites Ausführungsbeispiel der erfindungsgemäßen Löschvorrichtung 1. Die Speicherleuchtstoffplatte 3 ist hier nicht dargestellt. Dargestellt ist die Auflage 18, oberhalb der sich die Aufnahmeebene 7 zum Aufnehmen und Führen der Speicherleuchtstoffplatte 3 befindet. Der Reflektor 11 hat hier zwei Reflektorflächen 19 und 20. Die Reflektorflächen 19, 20 weisen jeweils eine Struktur auf, die im Wesentlichen einem unregelmäßigen Dreieck entspricht. Dabei sind die dreieckförmig strukturierten Reflektorflächen 19, 20 in Richtung der Aufnahmeebene 7 offen. Die Schnittpunkte der kurzen Seiten mit den langen Seiten der Reflektorflächen 19, 20 sind weiter von der Aufnahmeebene 7 entfernt als der parallel zur Aufnahmeebene 7 verlaufende Sockel 33. Der Sockel 33 ist somit näher an der Aufnahmeebene 7 angeordnet als ein Teil des Reflektors 11. Der Sockel 33 hat in Transportrichtung 6 eine Breite 41, die hier 42 mm beträgt.

Fig. 4 zeigt eine Draufsicht auf die Unterseite der Strahlungsquelle 8 gemäß Fig. 3. Zu sehen ist die parallele Anordnung der Leuchtdioden der beiden Leuchtdiodenzeilen 9, 10. Die Strahlungsquelle 8 erstreckt sich über eine Länge 42, die wengistens so groß ist wie die Längsausdehnung der Speicherleuchtstoffschicht 2. Fig. 5 zeigt zur weiteren Verdeutlichung eine perspektivische Darstellung der Löschvorrichtung 1 gemäß dem zweiten Ausführungsbeispiel nach den Fig. 3 und 4.

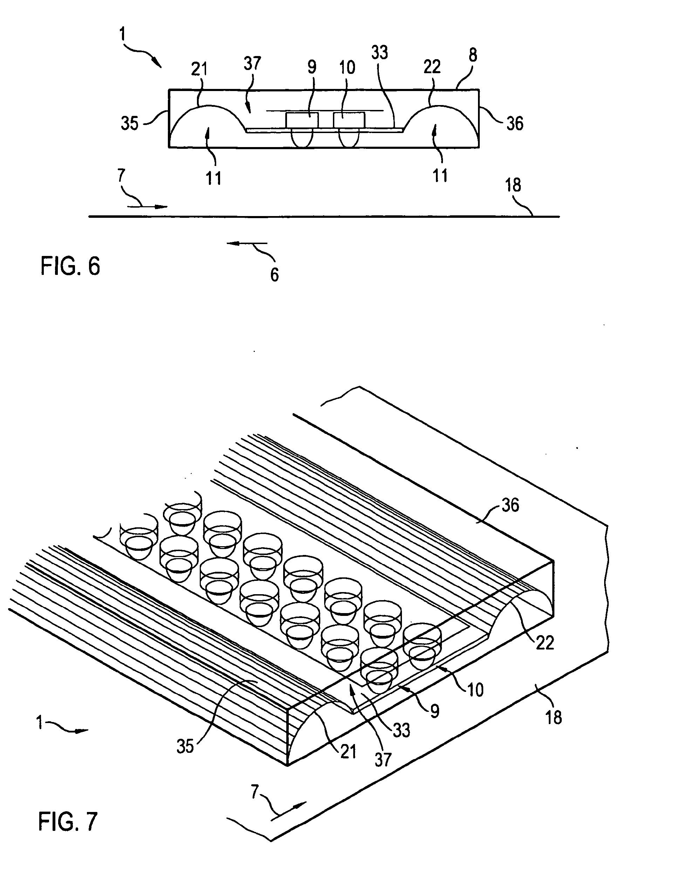

Fig. 6 zeigt ein drittes Ausführungsbeispiel der erfindungsgemäßen Löschvorrichtung 1. Die Strahlungsquelle 8 weist hier einen Reflektor 11 mit rinnenförmigen Reflektorflächen 21 und 22 auf. Die rinnenförmigen Reflektorflächen 21 und 22 sind dabei so ausgestaltet, dass sie ausgehend von ihren Verbindungsstellen mit dem Sockel 33 von der Aufnahmeebene 7 für die Speicherleuchtstoffplatte 3 weg gewölbt sind. In diesem Ausführungsbeispiel ist der Sockel 33 somit ebenfalls näher an der Aufnahmeebene 7 als ein Teil der Reflektorflächen 21, 22. Fig. 7 zeigt zur weiteren Verdeutlichung eine perspektivische Darstellung der Löschvorrichtung 1 gemäß dem dritten Ausführungsbeispiel nach Fig. 6.

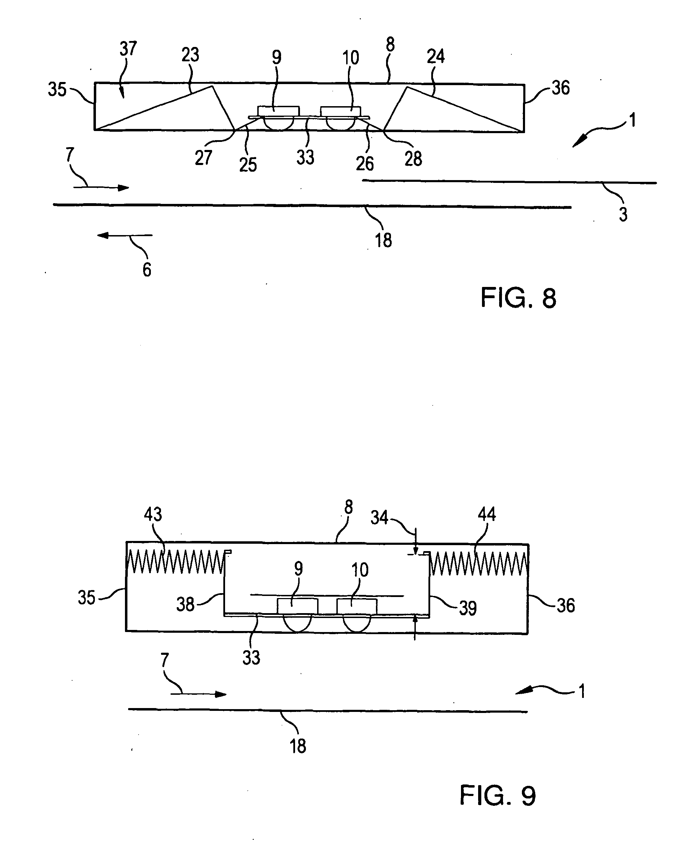

Fig. 8 zeigt ein viertes Ausführungsbeispiel der erfindungsgemäßen Löschvorrichtung 1. In der Fig. 8 ist die in die Löschvorrichtung 1 einfahrende Speicherleuchtstoffplatte 3 dargestellt. Der Sockel 33, auf dem die beiden Leuchtdiodenzeilen 9, 10 angeordnet sind, ist hier im Vergleich zum zweiten Ausführungsbeispiel nach Fig. 3 weiter von der Aufnahmeebene 7 weg versetzt. Der Reflektor 11 entspricht hier weitgehend demjenigen des zweiten Ausführungsbeispiels nach Fig. 3 und weist zwei Reflektorflächen 23 und 24 auf, die, in Transportrichtung 6 betrachtet, zu beiden Seiten des Sockels 33 angeordnet sind. Die Reflektorflächen 23, 24 weisen jeweils eine Struktur auf, die im Wesentlichen einem unregelmäßigen Dreieck entspricht, wie dies auch bei den Reflektorflächen 19, 20 der Fall ist. Zusätzlich zu dieser Dreiecksstruktur weisen die Reflektorflächen 23, 24 weitere Reflexionsflächen 25 und 26 auf. Diese weiteren Reflexionsflächen 25, 26 verlaufen ausgehend von der Aufnahmeebene 7 am nächsten liegenden Kanten 27 und 28 der Dreiecksstruktur schräg von der Aufnahmeebene 7 weg in Richtung des Sockels 33 und treffen schließlich auf diesen. Dadurch werden Verbindungen zwischen den Reflektorflächen 23, 24 und dem Sockel 33 hergestellt. Die Leuchtdiodenzeilen 9, 10 sind gegenüber den Kanten 27, 28 in einer Art Vertiefung angeordnet. Die Leuchtdiodenzeilen 9, 10 sind daher in dieser Ausführung besonders gut vor Strahlung geschützt, die von den Reflektorflächen 23, 24 in Richtung der Aufnahmeebene reflektiert wird. Der Sockel 33 ist allerdings auch in diesem vierten Ausführungsbeispiel näher an der Aufnahmeebene 7 platziert als ein Teil der Reflektorflächen 23, 24.

Fig. 9 zeigt ein fünftes Ausführungsbeispiel der erfindungsgemäßen Löschvorrichtung 1. Dargestellt ist die Auflage 18, oberhalb der sich die Aufnahmeebene 7 zum Aufnehmen und Führen der Speicherleuchtstoffplatte 3 befindet. Die Löschvorrichtung 1 enthält den Reflektor 11. Dieser hat hier zwei Reflektorflächen 43 und 44, die parallel zu der Aufnahmeebene 7 und der Auflage 18 verlaufen und zu beiden Seiten des Sockels 33 angeordnet sind. Die Reflektorflächen 43, 44 weisen jeweils eine Struktur auf, die hier im Wesentlichen dreieckförmig, nach Art von feinen Sägezähnen ausgestaltet ist. Mittels dieser Struktur ist ein retroreflektives Profil der Reflektorflächen 43, 44 realisiert. Dies bedeutet, dass die retroreflektiven Reflektorflächen 43, 44 vorteilhafterweise zumindest einen Teil der Löschstrahlung an Stellen der Speicherleuchtstoffschicht zurückreflektiert, an denen sie zuvor von der Speicherleuchtstoffschicht reflektiert wurde. Der Sockel 33 ist näher an der Aufnahmeebene 7 angeordnet als die Reflektorflächen 43, 44. Der Abstand 34 zwischen dem Sockel 33 und mit den Reflektorflächen 43, 44 wird mittels der Verbindungsflächen 38 und 39 überbrückt.

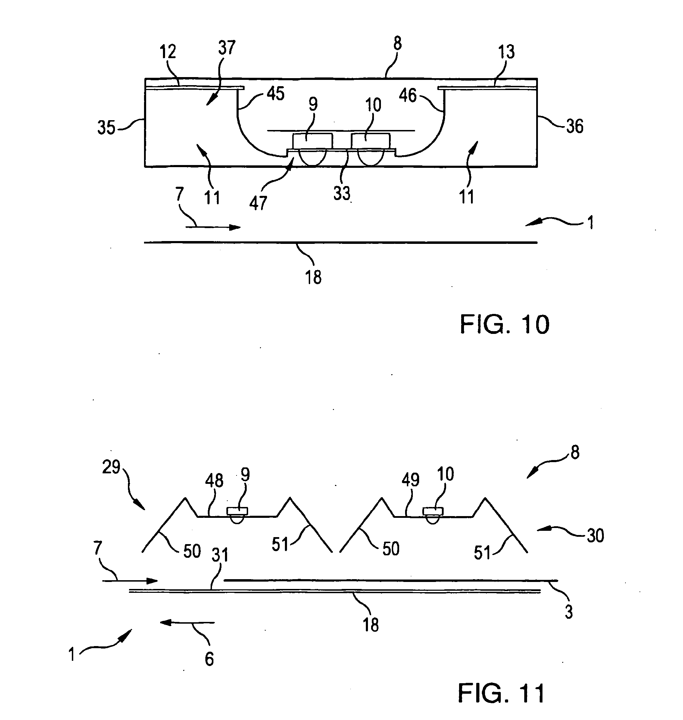

Fig. 10 zeigt ein sechstes Ausführungsbeispiel der erfindungsgemäßen Löschvorrichtung 1. Der Reflektor 11 weist hier, wie derjenige des ersten Ausführungsbeispiels nach Fig. 1, die ebenen Reflektorflächen 12, 13 auf, die zu beiden Seiten des Sockels 33 angeordnet sind. Der Sockel 33 ist näher an der Aufnahmeebene 7 angeordnet als die Reflektorebenen 12, 13. Der Sockel 33 ist über Verbindungsflächen 45 und 46 mit den Reflektorebenen 12, 13 verbunden. Im Gegensatz zu den ebenen und vertikal verlaufenden Verbindungsflächen 38, 39 des ersten Ausführungsbeispiels nach Fig. 1 sind die Verbindungsflächen 45, 46 in Richtung der Aufnahmeebene 7 gewölbt. Die beiden Leuchtdiodenzeilen 9, 10 sind zudem in einer Vertiefung 47 in dem Sockel 33 platziert.

Dadurch sind die Leuchtdiodenzeilen 9, 10 besonders gut gegen in sie hinein reflektierte Löschstrahlung geschützt.

Fig. 11 zeigt ein siebtes Ausführungsbeispiel der erfindungsgemäßen Löschvorrichtung 1, in welche die Speicherleuchtstoffplatte 3 einfährt. In diesem siebten Ausführungsbeispiel enthält die Strahlungsquelle 8 die zwei Leuchtdiodenzeilen 9, 10, die hier allerdings eigene Reflektoren aufweisen und auf voneinander getrennten Sockeln angeordnet sind. Die Leuchtdiodenzeile 9 ist auf einem Sockel 48 angeordnet und in einen Reflektor 29 integriert. Die Leuchtdiodenzeile 10 ist auf einem Sockel 49 angeordnet und in einen Reflektor 30 integriert. Die beiden Sockel 48, 49 und die beiden Reflektoren 29, 30 sind in Transportrichtung 6 voneinander beabstandet und getrennt. Dadurch treffen die von den Leuchtdiodenzeilen 9, 10 ausgegebenen Löschstrahlungen voneinander getrennt auf die in der Löschvorrichtung 1 befindliche Speicherleuchtstoffschicht 2 auf.

Die Leuchtdiodenzeilen 9, 10 geben Löschstrahlung in verschiedenen Wellenlängenbereichen aus. Die Leuchtdiodenzeile 9 gibt Löschstrahlung im blauen Wellenlängenbereich und die Leuchtdiodenzeile 10 im roten Wellenlängenbereich aus. Dadurch lässt sich vorteilhafterweise eine gute "Farbtrennung", und damit eine hohe Löscheffizienz, erreichen.

In Transportrichtung 6 der Speicherleuchtstoffplatte 3 trifft daher zuerst blaue und anschließend rote Löschstrahlung auf die Speicherleuchtstoffschicht 2. Ferner ist die Intensität der langwelligen, roten Löschstrahlung größer als die Intensität der kurzwelligen, blauen Löschstrahlung.

Der Intensitätsanteil der roten Löschstrahlung beträgt hier vorteilhafterweise ca. 66% und der Intensitätsanteil des blauen Löschlichts ca. 33%. Auf diese Weise ist eine besonders gute Löscheffizienz gewährleistet.

Die Reflektoren 29, 30 haben jeweils Reflektorflächen 50 und 51, die zu beiden Seiten der Sockel 48, 49 angeordnet sind. Die Reflektorflächen 50, 51 haben eine Form, die im Wesentlichen einem unregelmäßigen Dreieck entspricht. Die kurze Seite dieser Dreiecksform grenzt an den jeweiligen Sockel 48, 49 an und verläuft schräg von der Aufnahmeebene 7 weg nach außen. Die lange Seite dieser Dreiecksform verläuft ausgehend von dem Schnittpunkt mit der kurzen Seite schräg in Richtung der Aufnahmeebene 7 nach außen. Dadurch sind die dreieckförmigen Reflektorflächen 50, 51 in Richtung der Aufnahmeebene 7 offen. Die Schnittpunkte der kurzen Seiten mit den langen Seiten der Reflektorflächen 50, 51 sind weiter von der Aufnahmeebene 7 entfernt als die parallel zur Aufnahmeebene 7 verlaufenden Sockel 48, 49. Die Sockel 48, 49 sind somit näher an der Aufnahmeebene 7 angeordnet als ein Teil der Reflektoren 29, 30.

In einer bevorzugten Variante dieser Ausführung ist vorgesehen, dass die einander zugewandten, inneren Reflektorflächen 51 bzw. 50 der beiden Reflektoren 29 und 30 steiler verlaufen, d.h. einen kleineren Winkel gegenüber dem Lot auf die Speicherleuchtstoffplatte 3 einschließen, als die voneinander abgewandten, äußeren Reflektorflächen 50 bzw. 51. Hierdurch wird eine besonders gute "Farbtrennung" und damit eine besonders hohe Löscheffizienz erreicht.

## Patentansprüche

1. Vorrichtung (1) zum Löschen einer Speicherleuchtstoffschicht (2) mit
- einer Aufnahmeebene (7), in welcher die Speicherleuchtstoffschicht (2) liegt oder bewegt werden kann,
- einer Strahlungsquelle (8, 9, 10) zum Bestrahlen der Speicherleuchtstoffschicht (2) mit Löschstrahlung, welche zum Löschen der Speicherleuchtstoffschicht (2) geeignet ist, und
- einem Reflektor (11; 29, 30) zum Reflektieren von Löschstrahlung in Richtung auf die Aufnahmeebene (7), wobei der Reflektor (11; 29, 30) derart angeordnet und/oder ausgestaltet ist, dass er Löschstrahlung, welche von der Speicherleuchtstoffschicht (2) reflektiert wird, in Richtung auf die Speicherleuchtstoffschicht (2) reflektiert, **dadurch gekennzeichnet, dass**
- die Strahlungsquelle (8, 9, 10) auf einem Sockel (33; 48, 49) angeordnet ist, wobei der Sockel (33; 48, 49) näher an der Aufnahmeebene (7) angeordnet ist als wenigstens ein Teil des Reflektors (11; 29, 30).

2. Vorrichtung nach Anspruch 1, **dadurch gekennzeichnet, dass** der Sockel (33; 48, 49) in Richtung der Aufnahmeebene (7) reflektierend ausgestaltet ist.

3. Vorrichtung nach Anspruch 1 oder 2, **dadurch gekennzeichnet, dass** der Sockel (33) insbesondere in Richtung der Aufnahmeebene (7) gewölbt ausgestaltet ist.

4. Vorrichtung nach einem der vorhergehenden Ansprüche, **dadurch gekennzeichnet, dass** der Sockel (33) wenigstens eine Vertiefung (47) aufweist, in der die Strahlungsquelle (8) ausgebildet ist.

5. Vorrichtung nach einem der vorhergehenden Ansprüche, **dadurch gekennzeichnet, dass** der Sockel (33) näher als der gesamte Reflektor (11) an der Aufnahmeebene (7) angeordnet ist.

6. Vorrichtung nach einem der vorhergehenden Ansprüche, **dadurch gekennzeichnet, dass** der Reflektor (11) eine von der Aufnahmeebene (7) weg gewölbte Reflektorfläche (21, 22) aufweist.

7. Vorrichtung nach einem der vorhergehenden Ansprüche, **dadurch gekennzeichnet, dass** der Reflektor (11; 29, 30) eine ebene Reflektorfläche (12, 13) aufweist, die insbesondere parallel zur Aufnahmeebene (7) verläuft.

8. Vorrichtung nach einem der vorhergehenden Ansprüche, **dadurch gekennzeichnet, dass** der Reflektor (11) eine Reflektorfläche (19, 20; 23, 24; 43, 44) mit einer Struktur aufweist.

9. Vorrichtung nach Anspruch 8, **dadurch gekennzeichnet, dass** die strukturierte Reflektorfläche (43, 44) retroreflektiv ausgestaltet ist, so dass sie zumindest einen Teil der Löschstrahlung an Stellen der Speicherleuchtstoffschicht (2) zurückreflektiert, an denen sie von der Speicherleuchtstoffschicht (2) zuvor reflektiert wurde.

10. Vorrichtung nach einem der vorhergehenden Ansprüche, **dadurch gekennzeichnet, dass** ein Antrieb (5) zum Erzeugen einer Relativbewegung zwischen der Aufnahmeebene (7) und der Strahlungsquelle (8) vorhanden ist.

11. Vorrichtung nach Anspruch 10, **dadurch gekennzeichnet, dass** der Reflektor (11; 29, 30) wenigstens zwei Reflektorflächen (12, 13; 19, 20; 23, 24; 43, 44) aufweist, so dass der Reflektor (11; 29, 30), in Richtung (6) der Relativbewegung betrachtet, zu beiden Seiten der Strahlungsquelle (8) ausgebildet ist.

12. Vorrichtung nach Anspruch 11, **dadurch gekennzeichnet, dass** der Reflektor (11; 29, 30) in Richtung (6) der Relativbewegung spiegelsymmetrisch ausgestaltet ist, wobei eine Symmetrieachse (16) senkrecht zur Richtung (6) der Relativbewegung und, in Richtung (6) der Relativbewegung betrachtet, zentral durch die Strahlungsquelle (8) hindurch verläuft.

13. Vorrichtung nach einem der Ansprüche 10-12, **dadurch gekennzeichnet, dass** eine Ausbreitung (14) des Reflektors (11) in Richtung (6) der Relativbewegung wenigstens zehnmal so groß ist wie ein kleinster Abstand (15) des Reflektors (11) von der Aufnahmeebene (7).

14. Vorrichtung nach einem der Ansprüche 10-13, **dadurch gekennzeichnet, dass** die Strahlungsquelle (8) wenigstens zwei senkrecht zur Richtung (6) der Relativbewegung und parallel zu der Aufnahmeebene (7) verlaufende Zeilen (9, 10) mit Leuchtdioden aufweist.

15. Vorrichtung nach Anspruch 14, **dadurch gekennzeichnet, dass** die wenigstens zwei Zeilen (9, 10) mit Leuchtdioden in den Sockel (33) integriert sind und ein Abstand (17) zwischen den wenigstens zwei Zeilen (9, 10) kleiner oder gleich einem Abstand (32) der Leuchtdioden zu der Aufnahmeebene (7) ist.

16. Vorrichtung nach Anspruch 14, **dadurch gekennzeichnet, dass** den wenigstens zwei Zeilen (9, 10) mit Leuchtdioden jeweils ein eigener Reflektor (29, 30) und jeweils ein eigener Sockel (48, 49) zugeordnet sind und die Leuchtdioden der jeweiligen Zeilen (9, 10) eine Strahlung in einem anderen, insbesondere schmalbandigen, Wellenlängenbereich emittieren als die Leuchtdioden der anderen Zeilen (9, 10).

17. Vorrichtung nach Anspruch 16, **dadurch gekennzeichnet, dass** die wenigstens zwei Zeilen (9, 10) mit Leuchtdioden in Richtung (6) der Relativbewegung so hintereinander angeordnet sind, dass beim Ausführen der Relativbewegung zum Löschen der Speicherleuchtstoffschicht (2) eine kurzwellige Löschstrahlung vor einer langwelligen Löschstrahlung auf die Speicherleuchtstoffschicht (2) trifft.

18. Vorrichtung nach Anspruch 16 oder 17, **dadurch gekennzeichnet, dass** eine Intensität der langwelligen Löschstrahlung größer ist als eine Intensität der kurzwelligen Löschstrahlung.

19. Vorrichtung nach einem der Ansprüche 10-18, **dadurch gekennzeichnet, dass** eine dem Reflektor (11; 29, 30), in Richtung senkrecht zur Richtung (6) der Relativbewegung betrachtet, gegenüberliegende, weitere reflektierende Fläche (31) zum Reflektieren von Löschstrahlung vorhanden ist.

20. Vorrichtung nach Anspruch 19, **dadurch gekennzeichnet, dass** die weitere reflektierende Fläche (31) auf der dem Reflektor (11; 29, 30) abgewandten Seite der Aufnahmeebene (7) angeordnet ist.

21. Vorrichtung nach einem der vorhergehenden Ansprüche, **dadurch gekennzeichnet, dass** Stirnflächen (35, 36) und/oder Seitenflächen (37) des Reflektors (11; 29, 30) reflektierend ausgestaltet sind.

22. System mit einer Vorrichtung (1) nach einem der vorhergehenden Ansprüche und einer Speicherleuchtstoffschicht (2).

23. System nach Anspruch 22, **dadurch gekennzeichnet, dass** die Speicherleuchtstoffschicht (2) einen Reflexionsgrad für die Löschstrahlung von größer oder gleich 70%, insbesondere größer oder gleich 80%, aufweist.

## Claims

1. An apparatus (1) for deleting a storage phosphor layer (2) comprising
- a receiving plane (7) in which the storage phosphor layer (2) lies or can be moved,
- a radiation source (8, 9, 10) for irradiating the storage phosphor layer (2) with deleting radiation which is suitable for deleting the storage phosphor layer (2), and
- a reflector (11: 29, 30) for reflecting deleting radiation in the direction of the receiving plane (7),
the reflector (11; 29, 30) being arranged and/or designed such that it reflects deleting radiation which is reflected by the storage phosphor layer (2), in the direction of the storage phosphor layer (2),
**characterised in that**
- the radiation source (8, 9, 10) is disposed on a base (33; 48, 49), the base (33; 48, 49) being disposed closer to the receiving plane (7) than at least part of the reflector (11; 29, 30).

2. The apparatus according to Claim 1, **characterised in that** the base (33; 48, 49) is designed to be reflective in the direction of the receiving plane (7).

3. The apparatus according to Claim 1 or 2, **characterised in that** the base (33) is curved in form, in particular in the direction of the receiving plane.

4. The apparatus according to any of the preceding claims, **characterised in that** the base (33) has at least one indentation (47) in which the radiation source (8) is formed.

5. The apparatus according to any of the preceding claims, **characterised in that** the base (33) is disposed closer than the whole reflector (11) to the receiving plane (7).

6. The apparatus according to any of the preceding claims, **characterised in that** the reflector (11) has a reflector surface (21, 22) that curves away from the receiving plane (7).

7. The apparatus according to any of the preceding claims, **characterised in that** the reflector (11; 29, 30) has a level reflector surface (12, 13) which extends in particular parallel to the receiving plane (7).

8. The apparatus according to any of the preceding claims, **characterised in that** the reflector (11) has a reflector surface (19, 20; 23, 24; 43, 44) with a structure.

9. The apparatus according to Claim 8, **characterised in that** the structured reflector surface (43, 44) is designed to be retroreflective so that it reflects back at least part of the deleting radiation at points of the storage phosphor layer (2) at which it was previously reflected by the storage phosphor layer (2).

10. The apparatus according to any of the preceding claims, **characterised in that** a drive (5) is provided for producing a relative movement between the receiving plane (7) and the radiation source (8).

11. The apparatus according to Claim 10, **characterised in that** the reflector (11; 29, 30) has at least two reflector surfaces (12, 13; 19, 20; 23, 24; 43, 44) so that, as observed in the direction (6) of the relative movement, the reflector (11; 29, 30) is formed to both sides of the radiation source (8).

12. The apparatus according to Claim 11, **characterised in that** the reflector (11; 29, 30) is designed mirror-symmetrically in the direction (6) of the relative movement, an axis of symmetry (16) extending perpendicularly to the direction (6) of the relative movement and, as observed in the direction (6) of the relative movement, centrally through the radiation source (8).

13. The apparatus according to any of Claims 10-12, **characterised in that** a widening (14) of the reflector (11) in the direction (6) of the relative movement is at least ten times as great as a smallest distance (15) between the reflector (11) and the receiving plane (7)

14. The apparatus according to any of Claims 10-13, **characterised in that** the radiation source (8) has at least two lines (9, 10) extending perpendicularly to the direction (6) of the relative movement and parallel to the receiving plane (7).

15. The apparatus according to Claim 14, **characterised in that** the at least two lines (9, 10) are integrated with light emitting diodes into the base (33), and a distance (17) between the at least two lines (9, 10) is smaller than or equal to a distance (32) between the light emitting diodes and the receiving plane (7).

16. The apparatus according to Claim 14, **characterised in that** a specific reflector (29, 30) and a specific base (48, 49) are respectively assigned to the at least two lines (9, 10) with light emitting diodes, and the light emitting diodes of the respective lines (9, 10) emit a radiation in a different, in particular narrowband, wavelength range than the light emitting diodes of the other lines (9, 10).

17. The apparatus according to Claim 16, **characterised in that** the at least two lines (9, 10) with light emitting diodes are disposed one behind the other in the direction (6) of the relative movement such that when implementing the relative movement in order to delete the storage phosphor layer (2) shortwave deleting radiation strikes the storage phosphor layer (2) before a longwave deleting radiation.

18. The apparatus according to Claim 16 or 17, **characterised in that** an intensity of the longwave deleting radiation is greater than an intensity of the shortwave deleting radiation.

19. The apparatus according to any of Claims 10-18, **characterised in that** a further reflective surface (31) for reflecting deleting radiation is provided opposite the reflector (11; 29, 30), as observed in the direction perpendicular to the direction (6) of the relative movement.

20. The apparatus according to Claim 19, **characterised in that** the further reflective surface (31) is disposed on the side of the receiving plane (7) facing away from the reflector (11; 29, 30).

21. The apparatus according to any of the preceding claims, **characterised in that** faces surfaces (35, 36) and/or side surfaces (37) of the reflector (11; 29, 30) are designed to be reflective.

22. A system with an apparatus (1) according to any of the preceding claims and with a storage phosphor layer (2).

23. The system according to Claim 22, **characterised in that** the storage phosphor layer (2) has a degree of reflection for the deleting radiation of greater than or equal to 70%, in particular greater than or equal to 80%.

## Revendications

1. Dispositif (1) d'effacement d'une couche d'enregistrement luminescente (2) comportant :
- un plan de réception (7) dans lequel la couche d'enregistrement luminescente (2) repose ou peut être déplacée,
- une source de rayonnement (8, 9, 10) permettant d'exposer la couche d'enregistrement luminescente (2) à un rayonnement d'effacement adapté pour effacer la couche d'enregistrement luminescente (2), et
- un réflecteur (11 ; 29, 30) permettant de réfléchir le rayonnement d'effacement en direction du plan de réception (7), en sachant que
- le réflecteur (11 ; 29, 30) est disposé et/ou conçu de sorte que le rayonnement d'effacement réfléchi par la couche d'enregistrement luminescente (2) soit renvoyé en direction de la couche d'enregistrement luminescente (2), **caractérisé en ce que**
- la source de rayonnement (8, 9, 10) est disposée sur un support (33 ; 48, 49), le support (33 ; 48, 49) étant situé plus près du plan de réception (7) que ne l'est au moins une partie du réflecteur (11 ; 29, 30).

2. Dispositif selon la revendication 1, **caractérisé en ce que** le support (33 ; 48, 49) est conçu pour être réfléchissant dans la direction du plan de réception (7).

3. Dispositif selon la revendication 1 ou 2, **caractérisé en ce que** le support (33) est conçu bombé, notamment dans la direction du plan de réception (7).

4. Dispositif selon l'une des revendications précédentes, **caractérisé en ce que** le support (33) présente au moins un renfoncement (47) dans lequel est formée la source de rayonnement (8).

5. Dispositif selon l'une des revendications précédentes, **caractérisé en ce que** le support (33) est disposé plus près du plan de réception (7) que ne l'est le réflecteur (11) pris dans son intégralité.

6. Dispositif selon l'une des revendications précédentes, **caractérisé en ce que** le réflecteur (11) présente une surface de réflecteur (21, 22) bombée en s'écartant du plan de réception (7).

7. Dispositif selon l'une des revendications précédentes, **caractérisé en ce que** le réflecteur (11 ; 29, 30) présente une surface de réflecteur (12, 13) plane qui se prolonge notamment parallèlement au plan de réception (7).

8. Dispositif selon l'une des revendications précédentes, **caractérisé en ce que** le réflecteur (11) présente une surface de réflecteur (19, 20 ; 23, 24 ; 43, 44) disposant d'une structure.

9. Dispositif selon la revendication 8, **caractérisé en ce que** la surface de réflecteur (43, 44) structurée est conçue pour être rétroréfléchissante, de sorte qu'au moins une partie du rayonnement d'effacement soit rétroréfléchie en des emplacements de la couche d'enregistrement luminescente (2) où elle était précédemment réfléchie par la couche d'enregistrement luminescente (2).

10. Dispositif selon l'une des revendications précédentes, **caractérisé en ce qu'**il est prévu un dispositif d'entraînement (5) destiné à produire un mouvement relatif entre le plan de réception (7) et la source de rayonnement (8).

11. Dispositif selon la revendication 10, **caractérisé en ce que** le réflecteur (11 ; 29, 30) présente au moins deux surfaces de réflecteur (12, 13 ; 19, 20 ; 23, 24 ; 43, 44) de sorte que ledit réflecteur (11 ; 29, 30), considéré dans le sens (6) du mouvement relatif, est conçu des deux côtés de la source de rayonnement (8).

12. Dispositif selon la revendication 11, **caractérisé en ce que** le réflecteur (11 ; 29, 30) est de conception symétrique dans le sens (6) du mouvement relatif, un axe de symétrie (16) se prolongeant perpendiculairement au sens (6) du mouvement relatif en passant par le centre de la source de rayonnement (8), vu dans le sens (6) du mouvement relatif.

13. Dispositif selon l'une des revendications 10 à 12, **caractérisé en ce qu'**une étendue (14) du réflecteur (11) dans le sens (6) du mouvement relatif est au moins dix fois plus importante qu'un plus petit écart (15) existant entre le réflecteur (11) et le plan de réception (7).

14. Dispositif selon l'une des revendications 10 à 13, **caractérisé en ce que** la source de rayonnement (8) présente au moins deux rangées (9, 10) de diodes électroluminescentes disposées perpendiculairement au sens (6) du mouvement relatif et parallèlement au plan de réception (7).

15. Dispositif selon la revendication 14, **caractérisé en ce que** les au moins deux rangées (9, 10) de diodes électroluminescentes sont intégrées dans le support (33) et **en ce qu'**un écart (17) existant entre les au moins deux rangées (9, 10) est inférieur ou égal à un écart (32) existant entre les diodes électroluminescentes et le plan de réception (7).

16. Dispositif selon la revendication 14, **caractérisé en ce que** les au moins deux rangées (9, 10) de diodes électroluminescentes disposent chacune d'un réflecteur propre (29, 30) et d'un support propre (48, 49) et les diodes électroluminescentes des rangées (9, 10) respectives émettent un rayonnement dans une plage de longueurs d'ondes différente, en étant notamment à bande étroite, de celle des diodes électroluminescentes des autres rangées (9, 10).

17. Dispositif selon la revendication 16, **caractérisé en ce que** les au moins deux rangées (9, 10) de diodes électroluminescentes sont agencées les unes derrière les autres dans le sens (6) du mouvement relatif de sorte que la couche d'enregistrement luminescente (2) subissant le mouvement relatif destiné à effacer ladite couche d'enregistrement luminescente (2) soit tout d'abord soumise à un rayonnement d'effacement à ondes courtes puis à un rayonnement d'effacement à ondes longues.

18. Dispositif selon la revendication 16 ou 17, **caractérisé en ce qu'**une intensité du rayonnement d'effacement à ondes longues est plus élevée qu'une intensité du rayonnement d'effacement à ondes courtes.

19. Dispositif selon l'une des revendications 10 à 18, **caractérisé en ce qu'**il est prévu une surface réfléchissante supplémentaire (31) en regard du réflecteur (11 ; 29, 30), considéré dans la direction perpendiculaire au sens (6) du mouvement relatif, qui est destinée à réfléchir le rayonnement d'effacement.

20. Dispositif selon la revendication 19, **caractérisé en ce que** la surface réfléchissante supplémentaire (31) est agencée sur la face du plan de réception (7) qui est détournée du réflecteur (11 ; 29, 30).

21. Dispositif selon l'une des revendications précédentes, **caractérisé en ce que** des surfaces frontales (35, 36) et/ou latérales (37) du réflecteur (11 ; 29, 30) sont conçues pour être réfléchissantes.

22. Système comportant un dispositif (1) selon l'une des revendications précédentes et une couche d'enregistrement luminescente (2).

23. Système selon la revendication 22, **caractérisé en ce que** la couche d'enregistrement luminescente (2) présente un degré de réflexion concernant le rayonnement d'effacement qui est supérieur ou égal à 70 %, notamment supérieur ou égal à 80 %.
